# EUROPEAN PATENT SPECIFICATION

(11) **EP 0 894 146 B1**
(45) Date of publication and mention of the grant of the patent: **01.10.2003**
(21) Application number: 97915776.5
(22) Date of filing: 25.03.1997
(51) Int. Cl.: C12Q 1/68, C12N 5/00, G01N 33/543

(54) **IMMUNO-MAGNETIC CELL SEPARATION USED IN IDENTIFICATION OF GENES ASSOCIATED WITH SITE-PREFERENCED CANCER METASTASIS FORMATION**
IMMUNO-MAGNETISCHE ZELLTRENNUNG ZUR ERKENNUNG VON KREBSMETASTASENBILDUNG-ASSOZIERTEN GENEN
PROCEDE IMMUNO-MAGNETIQUE DE SEPARATION CELLULAIRE UTILISE POUR L'IDENTIFICATION DE GENES ASSOCIES A LA FORMATION DE METASTASES CANCEREUSES A PREFERENCE DE SITE

(30) Priority: 26.03.1996 NO 961221
(43) Date of publication of application: 03.02.1999
(73) Proprietor: FODSTAD, Oystein, 0383 Oslo (NO)
(72) Inventor: FODSTAD, Oystein, N-0383 Oslo (NO); ENGEBRATEN, Olav, N-1470 Lorenskog (NO); REE, Anne, Hansen, N-1344 Haslum (NO); HOVIG, Johannes, Eivind, N-0851 Oslo (NO)
(74) Representative: Tombling, Adrian George
(86) International application number: NO9700083
(87) International publication number: WO97036004

(56) References cited:
- WO-A-94/07139
- WO-A-95/24648
- NATIONAL LIBRARY OF MEDICINE, File Medline, Medline Accession No. 95268055, VAN EPPS DE et al., "Harvesting, Characterization and Culture of CD34+ Cells from Human Bone Marrow, Peripheral Blood and Cord Blood"; & BLOOD CELLS, 1994;20(2-3):411-23.
- DIALOG INFORMATION SERVICE, file 154, Medline, Dialog Accession No. 07911215, Medline Accession No. 94231000, WIXLER V. et al., "Isolation and Quantification of Class 1 MHC Gene Mutants in Mouse T Cells by Immunoselection with a Magnetic Cell Sorter (MACS)"; & J. IMMUNOL. METHODS, 2 May 1994, 171(1), p121-30.
- DIALOG INFORMATION SERVICE, File 154, Medline, Dialog Accession No. 06887713, Medline Accession No. 92084142, KAPPES D.J. et al., "A Novel Method for Generating Stabel High-Level Transfectants Involving Direct Immunomagnetic Selection for Cell-Surface Epitopes: Expression of HLA Class-II Genes in HeLa Cells"; & GENE, 15 Dec. 1991, 108(2), 245-52.
- DIALOG INFORMATION SERVICE, File 159, Cancerlit, Dialog Accession No. 01195522, Cancerlit Accession No. 96133084, YAREMKO M.L. et al., "Immunomagnetic Separation Can Enrich Fixed Solid Tumors for Epithelial Cells"; & AM. J. PATHOL., 148(1):95-104, 1996.
- NATIONAL LIBRARY OF MEDICINE, File Medline, Medline Accession No. 95325659, GRIWATZ C. et al., "An Immunological Enrichment Method for Epithelial Cells from Peripheral Blood"; & J. IMMUNOL. METHODS, 1995, Jun. 28;183(2):251-65.
- DIALOG INFORMATION SERVICE, File 159, Cancerlit, Dialog Accession No. 01193425, Cancerlit Accession No. 96709393, REE A.H. et al., "Identification of Site Specific Gene Expression in Metastatic Breast Cancer Cells (Meeting Abstract)"; & PROC. ANNU. MEET. AM. ASSOC. CANCER RES., 37:A607, 1996.
- DIALOG INFORMATION SERVICE, File 159, Cancerlit, Dialog Accession No. 01192976, Cancerlit Accession No. 96653514, BRANDT B. et al., "Isolation of Single Cancer Cells and Coherent Cell Sheets from Peripheral Blood of Cancer Patients with Reference to Shed Cytokeratin 8/18 and ErbB-2 Oncoprotein Fragments (Meeting Abstract)"; & PROC. ANNU. MEET. AM. ASSOC. CANCER RES., 37:A555, 1996.
- DIALOG INFORMATION SERVICE, File 159, Cancerlit, Dialog Accession No. 00864158, Cancerlit Accession No. 92257480, GAZITT Y. et al., "Expression of N-Myc and MDR-1 Proteins in Newly Established Neuroblastoma Cell Lines: A Study by Immunofluorescence Staining and Flow Cytometry"; & CANCER RES., 52(10):2957-65, 1992.
- Pei et al., "Expression of 32-kDa laminin-binding protein mRNA in colon cancer tissues", J.Surg. Res. 61/1, 1996, pp. 120-6
- Dummer et al., "Interleukin-10 production in malignant melanoma: preferential detection of IL-10-secreting tumor cells in metastatic lesions", Int.J.Cancer 66/5, 1996, pp. 607-10
- Zeng et al., "Distinct pattern of matrix metalloproteinase 9 and tissue inhibitor of metalloproteinase 1 mRNA expression in human colorectal cancer and liver metastases", Brit.J.Cancer 72/3, 1995, pp. 575-82
- Kuranami et al., "Differential expression of protein kinase C isoforms in human colorectal cancers", J.Surg.Res. 58/2, 1995, pp. 233-9
- Mafune et al., "Expression of a Mr 32,000 laminin-binding protein messenger RNA in human colon carcinoma correlates with disease progression", Cancer Research 50/13, 1990, pp. 3888-91

## Description

The object of the present invention is to provide a novel approach for detecting new genes with site-specific expression patterns in tumor cells residing in different tissues.

It is well known that many cancer types show typical patterns of spread, in that metastases appear preferably in certain tissues or organs, often in an orderly fashion. Thus, breast cancer metastases usually first develop in axillary lymph nodes, whereas bone marrow/bone metastases represent the first and most common (50%) site of distant spread. Of other tissues, liver, lung and the central nervous system (up to 20%) become hosts of breast cancer metastases. Similarly, prostate cancer gives skeletal metastases; colon cancer spreads to lymph nodes and liver; osteosarcoma to lung; and malignant melanoma to lymph nodes, liver, lung and brain. Very little is known about the factors that determine such tissue-preferenced cancer spread, but specific characteristics of the tumor cells are certainly involved, by e.g. enabling the tumor cells to home in the target organ, move to and invade the host tissue, respond to local growth factors, induce angiogenesis, or react in hitherto unknown ways. These characteristics must be associated with expression of specific proteins expressed by known or unknown genes. To identify such genes may, therefore, be of great importance in the understanding of the mechanisms of metastasis and thereby provide new leads or clues for diagnosis and therapy.

Several methods are being used in the search for genes that are highly expressed in certain populations of cells and not in other, including procedures such as subtractive hybridization cloning and the use of the differential display approach. Most such cloning projects have involved the use of *in vitro* cell lines or clones as starting material. However, it is known that cell lines may differ significantly from the tumor cells from which they originated, and that *in vitro* culture conditions may up- or down-regulate the expression of genes involved in deciding the ability of the cells to invade extracellular matrices and stromal tissue as well as their overall metastatic capacity.

A logical alternative to cell lines for comparing the expression of gene transcripts or proteins of interest would be to use specimens of tumor tissue from primary tumors and metastases from patients. Such an approach does, however, include several possibilities for error and also technical difficulties. When using tumor cells from different patients the expression of genes characteristic of each individual must be subtracted before the patterns of gene expression related to the objective of the study are compared. This adds to the immense complexity of such gene cloning. It would be advantageous, therefore, to be able to compare expression patterns in cancer cells obtained from tumor manifestations located at different sites in one and the same individual. This may be possible by collecting tumor tissue from both the primary tumor and overt metastases detected and removed at surgery, and/or by surgery or biopsy of recurrent disease, or from secondary tumors in patients with progressive disease who have or have not received other treatment modalities after primary surgery.

In any gene cloning project it is important to work with as pure populations of target cells as possible, attempting to avoid irrelevant signals from non-target cells blurring the expression patterns to be compared. In specimens from solid tumors this is difficult to achieve, as in surgical specimens and biopsies the tumor cells will be mixed with normal fibrous tissue, including stromal and endothelial cells, that conventional methods of tissue preparation cannot satisfactorily remove. In hematological cancers, the malignant cells share determinants of a corresponding subpopulation of normal cells, preventing separation of the two types of cells.

In attempts to identify genes that are involved in the early stages of tumor dissemination, it would be important to obtain tumor cells from the relevant sites when the size of the secondary tumors is small, or if possible even from subclinical tumor foci or cells. One example would be malignant cells present in blood or in bone marrow before conventional diagnostic measures can demonstrate solid manifestation of metastasis. Another example would be cancer cells present in cerebrospinal fluid, in urine, or in effusions in pleural and abdominal cavities before conventional morphological procedures can detect such cells. Moreover, at primary surgery or if suspected for metastatic spread, lymph nodes are often removed because they are enlarged. Morphological examination may, however, still be negative in cases where a limited number of tumor cells still might be present. In all these examples the present invention describes a means of detecting and selecting the target tumor cells for gene cloning purposes.

In addition to using cancer cells obtained from different tissues or organs in patients, the invention also describes another way of obtaining metastatic human tumor cells for use in the search for genes with site-specific expression. Cells from several human tumors have been grown *in vitro* or in immunodeficient animals *in vivo,* and such cells have been used to establish experimental metastasis models or models in which the cells can be grown orthotopically, i.e. in the clinically relevant tissues of origin; malignant melanoma in the skin; osteosarcoma in bone; colorectal cancer in the bowel wall; breast cancer in the mammary fat pad, etc. In experimental metastasis models different patterns of tumor spread can be seen, depending on the cell line, the route of cell injection, and the type of host used. Commonly the metastasis patterns simulate that of the corresponding tumor type in the clinic. By using such models it becomes possible also to obtain tumor cells from metastatic sites not usually accessible in patients, such as the spinal cord and the brain tissue. Again, for gene cloning purposes it would be important to select the human tumor cells from the animal cells to avoid problems with genes expressed in the normal host cells.

Previously it has been impossible to perform meaningful gene cloning experiments on specimens of solid tumors and metastases and on malignant cells in blood and bone marrow with the object of identifying genes with site-specific expression. This is because a considerable fraction of solid tumors and metastases is not malignant cells but connective tissue supporting and growing in between the malignant cells, and which also contains blood vessels that provide the necessary supply of nutritional factors and oxygen to the tumor. It has not been regarded as possible to adequately separate tumor cells from the normal cells without including an intermediate step of culturing the cells *in vitro,* involving manipulations to get rid of the normal cells. However, such *in vitro* cultivation would result in a selection of subpopulation of tumor cells in an environment quite different from the situation *in vivo*, thereby inducing significant changes in gene expression patterns. Therefore, for the purpose of identifying genes associated with the metastatic process, cultivation of tumor cells from solid metastases cannot be used. Moreover, it has not been regarded of interest to persons known in the field of gene cloning to compare gene expression patterns in malignant cells in untreated solid primary and metastatic tumors. Furthermore, in samples of blood and bone marrow tumor cells, if at all present, constitute a very low fraction of the total number of nucleated cells, and the malignant cells in blood and bone marrow have not been regarded of interest for gene cloning attempts. This is because the tumor cells could not be adequately separated from normal cells, and importantly also because people known in the art of gene cloning have not expected that such malignant cells were sufficiently different in gene expression patterns from those in the parent tumors.

Cancer cells isolated from human tumors metastasizing to different and clinically relevant target tissues and organs in immunodeficient animals have not been used in attempts to identify tumor-associated genes with site-specific expression, simply because such human tumor models are very rare, but importantly also because of the lack of methods to obtain pure populations of cancer cells.

The object of the present invention is therefore to provide a method for identifying genes differentially expressed between target cells obtained from tumour manifestations located at different sites in one and the same individual.

This object has been obtained by the present invention characterized by the enclosed claims.

Since the object of the invention is to identify genes expressed specifically during early stages of tumor dissimulation, the solid tumors and metastases should be small in size, and the malignant cells in blood and bone marrow should represent so-called micrometastatic disease, i.e. a limited a number of tumor cells should be present. If possible, such tumor cells should be collected even in cases where said cells cannot be detected by conventional morphological examinations or with diagnostic procedures such as radiology and magnetic resonance imaging. Evidently, since such cells have not been recognizable they have not been of interest for gene cloning purposes. The use of immunomagnetic techniques permits isolation of said malignant cells even when present in low numbers. Various methods for amplifying DNA and RNA sequences make gene cloning possible on such low numbers of malignant cells, provided that this cell population is sufficiently pure.

The positive selection of target tumor cells can be obtained by the use of per se known techniques, as described in patent application PCT/NO93/00136 (WO 94/07139) and in PCT/N095/00052. Since in the present case the purity of the final target cell population is important, the immunomagnetic selection process may be performed more than once, or may be performed as a combination of positive (with target-cell recognizing monoclonal antibodies) and negative (with antibodies that bind to unwanted cells) selection. These techniques have been successfully used to isolate target cells from blood, bone marrow, malignant effusions, and from single cell suspensions prepared from solid tumor tissue of primary tumors and lymph node and other metastases. Similarly, the selection of human malignant cells from normal stromal or hematopoietic cells in animal hosts has also been demonstrated, even in cases where the tumor cells resided in bone, bone marrow, the spinal cord or in brain tissue. Since one objective would be to search for genes with products involved in the early stages of metastasis formation it may happen that only relatively few tumor cells can be obtained, the purity of which is particularly important. With this approach it is possible to obtain a better purified target cell population from patients and animal hosts than by any other known technique, providing unique possibilities for cloning genes with site-specific expression.

The next step of the invention involves the use of known gene cloning procedures, such as the use of the differential display procedure first described by Liang, A. and Pardee, A.B. (Science, Vol 257, 967-971, 1992). In this method, polymerase chain reactions are performed with enzymes and primers that give reverse transcription and random amplification of gene transcripts present in the target cells. Resulting cDNA fragments from the cell populations to be compared are thereafter studied on a sequencing gel and the site-specific fragments extracted and sequenced. Gene fragments of interest can then be further studied, including examination of their expression patterns in material similar to that used for cloning. Again, having access to purified tumor cell populations without irrelevant non-target cells interfering with the results is very important. The possibility of using human tumor cells isolated from metastasis models in immunodeficient animals is advantageous also because the models provide a reliable and continuous source of target tumor cells for extended studies. It is also important that the target cells, either from patients or from animal models, are isolated and treated for DNA and RNA studies directly and very rapidly to avoid unwanted alterations in gene expression not related to the objective of identifying genes with site-specific expression.

Different methods used for cloning new genes have their inherent limitations. The differensial display-methods based on polymerase chain reaction techniques may suffer from problems related to representativity and reproducability. With our approach we have consequently included steps aimed at minimizing such problems. This has been achieved mainly by the use of the immunobead selection technique which makes it possible to study specifically biologically representative cells, not only for the first differential display step, but also in the steps where the candidate genes are examined for expression levels in relevant cells and tissues.

### Example 1:

Tumor cells from the primary tumor and axillary lymph nodes from a breast cancer patient were prepared by physical and enzymatic methods to obtain a single cell suspension. A bone marrow sample (50 ml) was aspirated and a peripheral blood sample was obtained by venous puncture, and the mononuclear cells from both occasions were isolated on Lymphoprep (Nycomed Pharma, Oslo, Norway). The cell suspensions were independently incubated with the MOC-31 and BM7 monoclonal antibodies, recognizing a pan-epithelial antigen and the MUC-1 gene product, respectively. After washing and incubation Dynabeads M-450 SAM SD (Dynal, Oslo, Norway), which bind to the Fc-region of the primary antibodies, were added. Tumor cells with bound antibody-Dynabeads M-450 SAM SD complex could thereafter be isolated from the normal mononuclear cells by the use of a strong magnet. The nature of the selected cells was confirmed microscopically. From the different cell populations RNA was extracted and the material subjected to the differential display cloning procedure (Liang and Pardee, 1992), in which partial cDNA-sequences from mRNA subpopulations obtained by reverse transcription were amplified by polymerase chain reaction. Comparison of cDNA-fragments from the various tumor cell populations were compared on a sequencing gel, and fragments specifically expressed in cells obtained from one of the sites were extracted and sequenced. Among a number of interesting gene sequences with specific expression either in tumor cells isolated, with our magnetic immunobead technology, from bone marrow or in tumor cells immunomagnetically isolated from lymph node metastases, we have found one cell cycle related transcription factor, one oncogene product, in addition to genes not yet identified. The expression of the two identified gene sequences in biologically relevant model systems and clinical material is presently being analyzed.

### Example 2:

In this example, cells from a model for experimental metastasis of a human breast cancer were used. In athymic, nude rats injection of MA-11 human breast cancer cells into the cisterna magna (CM) results in leptomeningeal spread and growth of the malignant cells. Moreover, MA-11 cells injected into the left cardiac ventricle (LV) form metastases in the spinal cord which result in the development of hind leg paralysis in the animals after approximately 35 days. Tumor cells from both locations were obtained by mincing the host tissue and preparing single cell suspensions, and the immunobead technique described under example 1 was used to positively select the malignant cells. Thereafter, RNA extraction of cells from the two sites, together with *in vitro* cultured MA-11 cells was performed and the material similarly subjected to the differential display cloning procedure as already described. In addition, mRNA extracted from relevant normal tissues in the rats were included as controls. It should be noted that the MA-11 cell line was established from micrometastatic tumor cells isolates with the immunomagnetic method from the bone marrow of a stage II breast cancer patient. Several candidate fragments specific for the cells growing in the leptomeninges or as metastases to the spinal cord have been detected. Sequence analysis showed that these fragments represent both novel and known genes. Among the fragments confirmed as differentially expressed in MA-11 cells selected from metastatic cell, four candidate genes have so far been examined in more detail. Two of these, termed LV1 and LV12 are particularly interesting. LV1 shows very high expression selectivity in tumor cells from spinal cord metastases, whereas LV12 is donwregulated in leptomeningeally growing cells. LV1 is found to be upregulated in a number of cell lines known to have a high capacity to form experimental metastasis in immunodeficient animals. In a panel of primary tumours from breast cancer pateients, low expression of LV12 was related to short survival of the corresponding patients. Altogether the results show that LV1 mRNA seems to be upregulated in highly metastatic cells, and LV12 mRNA level was inversely correlated to progression and metastasis of breast cancer cells. Both genes are novel. In addition, a third promising candidate gene, CM13, is also a novel gene upregulated in tumor cells growing in the leptomeninges. Several other candidates are still to be further analyzed. For both LV1 and LV12 cDNA full length cloning has been initiated.

### Examples 3:

In a similar model to that described in example 2, MT1 human mammary cancer cells give rise to growth in the leptomeninges after CM injection, whereas LV injected cells metastasize to the bone marrow and bone in the vertebra of the spine and in the long bones. Cells from these two locations as well as *in vitro* grown cells have been isolated and RNA from the different cell populations has been extracted. Similar cloning procedures to those described in example 2 can be applied to this material.

### Example 4:

Tumor cells isolated by the technique described in example 1 have been isolated from other breast cancer patients, from patients with colorectal cancers where the tumor cells were isolated from primary and recurrent tumors, from lymph nodes and liver metastasis, and blood and bone marrow samples, and from prostate cancer patients with cells selected from primary or recurrent tumor specimens, from lymph nodes in addition to specimens from blood and bone marrow. The isolated cells can be used for gene cloning purposes.

In addition to the use of the material described in examples 1-4 for gene cloning, it can be used for examining the expression patterns of all gene sequences of sufficient promise.

## Claims

1. Method for identifying genes differentially expressed between target cells obtained from tumour manifestations located at different sites in one and the same individual,
**characterized in that** the target cells initially are detected and isolated by repeated immuno-magnetic procedures in order to obtain up to 100% specific target cells before exposing the said target cells to known gene cloning procedures, comparing levels of mRNA expressions in the target cells isolated from different tissues and identifying genes differentially expressed between the different tissues.

2. Method according to claim 1, wherein the levels of mRNA expressions are compared by comparing gene expression patterns of the target cells isolated from the different tissues.

3. Method according to claim 1 or claim 2, wherein the gene cloning procedures comprise random amplification of gene transcripts present in the target cells.

4. Method according to claim 1, claim 2 or claim 3,
**characterized in that** the used target cells are malignant cells obtained from solid primary or recurrent tumours; and/or from metastases from such tumours to lymph nodes; and/or blood; and/or bone marrow; and/or bone tissue; and/or liver; and/or lungs; and/or central nervous system; and/or malignant pleural effusions and ascites, urine; and/or cerebral spinal fluid; and/or other organ sites.

5. Method according to any one of claims 1 to 4,
**characterized in that** the malignant cells are isolated from single cell suspensions prepared from solid tumour manifestations; and/or from mononuclear cell fractions obtained from bone marrow or blood samples; and/or from cells present in other body fluids.

6. Method according to any one of claims 1 to 5,
**characterized in that** RNA and/or DNA are extracted from the isolated cells.

7. Method according to claim 6,
**characterized in that** the extracted nucleic acids are used for gene cloning purposes.

8. Method according to any one of the previous claims,
**characterized in that** the said gene cloning method is the differential display or subtractive hybridization approaches, or any other procedure that can be used to identify genes with differential expression.

9. Method according to claim 8,
**characterized in that** amplified cDNAs obtained from malignant cells selected from different sites are studied and compared on sequencing gels, and where those with interesting site-specific or site-preferenced patterns are sequenced and identified.

10. Method according to claim 9,
**characterized in that** the expression patterns of identified gene sequences are studied on material obtained from all relevant tumour cell sites.

11. Use of method according to claim 1, to obtain specific gene sequences and their expression products in target cells present in cell environments different or not from their origin.

## Patentansprüche

1. Verfahren zur Identifizierung von Genen, die differentiell zwischen Zielzellen exprimiert werden, die aus Tumormanifestationen gewonnen werden, die an verschiedenen Stellen in ein und dem selben Individuum lokalisiert sind, **dadurch gekennzeichnet, dass** die Zielzellen anfänglich durch wiederholte immunmagnetische Verfahren nachgewiesen und isoliert werden, um bis zu 100 % spezifische Zielzellen zu erhalten, bevor die besagten Zielzetlen bekannten Genklonierungsverfahren ausgesetzt werden, bei denen die Mengen der mRNA-Expressionen in den Zielzellen verglichen werden, die aus verschiedenen Geweben isoliert wurden, und Gene identifiziert werden, die differentiell zwischen den verschiedenen Geweben exprimiert werden.

2. Verfahren gemäß Anspruch 1, worin die Mengen der mRNA-Expressionen durch Vergleichen der Genexpressionsmuster der Zielzellen, die aus verschiedenen Geweben isoliert werden, verglichen werden.

3. Verfahren gemäß Anspruch 1 oder Anspruch 2, worin die Genklonierungsverfahren die zufällige Amplifikation von Gentranskripten, die in den Zielzellen vorhanden sind, umfassen.

4. Verfahren gemäß Anspruch 1, Anspruch 2 oder Anspruch 3, **dadurch gekennzeichnet, dass** die verwendeten Zielzellen maligne Zellen sind, die aus festen primären oder wiederkehrenden Tumoren und/oder aus Metastasen von solchen Tumoren in Lymphknoten und/oder Blut und/oder Knochenmark und/oder Knochengewebe und/oder Leber und/oder Lungen und/oder dem zentralen Nervensystem und/oder malignen pleuralen Effusionen und Ascites, Urin und/oder zerebraler Spinalflüssigkeit und/oder anderen Organstellen gewonnen werden.

5. Verfahren gemäß jeglichem der Ansprüche 1 bis 4, **dadurch gekennzeichnet, dass** die malignen Zellen aus einzelnen Zellsuspensionen isoliert werden, die aus festen Tumormanifestationen hergestellt werden und/oder aus mononuklearen Zellfraktionen, die aus Knochenmark oder Blutproben und/oder aus Zellen, die in anderen Körperflüssigkeiten vorhanden sind, gewonnen werden.

6. Verfahren gemäß einem jeglichen der Ansprüche 1 bis 5, **dadurch gekennzeichnet, dass** RNA und/oder DNA aus den isolierten Zellen extrahiert wird.

7. Verfahren gemäß Anspruch 6, **dadurch gekennzeichnet, dass** die extrahierten Nukleinsäuren für Genklonierungszwecke verwendet werden.

8. Verfahren gemäß einem jeglichen der vorangegangenen Ansprüche, **dadurch gekennzeichnet, dass** das besagte Genklonierungsverfahren die Differential-Display- oder subtrahierende Hybridisierungsansätze oder jegliches andere Verfahren ist, das verwendet werden kann, um Genes mit differentieller Expression zu identifizieren.

9. Verfahren gemäß Anspruch 8, **dadurch gekennzeichnet, dass** die amplifizierten DNA's, die aus malignen Zellen gewonnen werden, die aus verschiedenen Stellen ausgewählt werden, auf Sequenziergelen untersucht und verglichen werden, und wobei solche mit interessanten positions-spezifischen oder positionsbevorzugten Mustern sequenziert und identifiziert werden.

10. Verfahren gemäß Anspruch 9, **dadurch gekennzeichnet, dass** die Expressionsmuster der identifizierten Gensequenzen auf Material untersucht werden, das aus allen relevanten Tumor-Zellstellen gewonnen wird.

11. Verfahren gemäß Anspruch 1, um spezifische Gensequenzen und deren Expressionsprodukte in Zielzellen zu gewinnen, die in Zellumgebungen vorhanden sind, die sich von ihrem Ursprung unterscheiden oder nicht.

## Revendications

1. Procédé d'identification de gènes s'exprimant différemment entre des cellules cibles obtenues à partir de manifestations tumorales localisées dans différents sites dans un seul et même individu,
**caractérisé en ce que** les cellules cibles sont initialement détectées et isolées par des procédures immuno-magnétiques répétées afin d'obtenir jusqu'à 100% de cellules ciblée spécifiques avant exposition desdites cellules cibles aux procédures connues de clonage de gènes, comparaison des niveaux d'expression de l'ARNm dans les cellules cibles isolées de différents tissus et identification des gênes s'exprimant différemment entre les différents tissus.

2. Procédé selon la revendication 1, dans lequel les niveaux d'expression de l'ARNm sont comparés en comparant les profile d'expression des gênes des cellules cibles isolées des différents tissus.

3. Procédé selon la revendication 1 ou la revendication 2. dans lequel les procédures de clonage de gênes comprennent l'amplification aléatoire des produits de la transcription des gênes présents dans les cellules cibles.

4. Procédé selon la revendication 1, la revendication 2 ou la revendication 3,
**caractérisé en ce que** les cellules cibles utilisées sont des cellules cancéreuses obtenues à partir de tumeurs solides primaires ou récurrentes ; et/ou de métastases de telles tumeurs vers les ganglions lymphatiques ; et/ou le sang ; et/ou la moelle osseuse ; et/ou les tissus osseux ; et/ou le foie ; et/ou les poumons ; et/ou le système nerveux central ; et/ou les épanchements pleuraux et ascites cancéreux, l'urine ; et/ou le fluide cérébro-spinal ; et/ou les autres sites d'organes.

5. Procédé selon l'une quelconque des revendications 1 à 4,
**caractérisé en ce que** les cellules cancéreuses sont isolées de suspensions d'une cellule unique préparées à partir de manifestations tumorales solides ; et/ou à partir de fractions de cellules mononucléaires obtenues à partir d'échantillons de moelle osseuse ou de sang ; et/ou à partir de cellules présentes dans d'autres fluides du corps.

6. Procédé selon l'une quelconque des revendications 1 à 5,
**caractérisé en ce que** l'ARN et/ou l'ADN sont extraits à partir des cellules isolées.

7. Procédé selon la revendication 6,
**caractérisé en ce que** les acides nucléiques extraits sont utilisés à des fine de clonages de gênes.

8. Procédé selon l'une quelconque des revendications précédentes,
**caractérisé en ce que** ledit procédé de clonage des gènes est l'affichage différentiel ou les approches d'hybridation souatractive, ou toute autre procédure qui peut être utilisée pour identifier les gènes avec une expression différentielle.

9. Procédé selon la revendication 8,
**caractérisé en ce que** les ADNc amplifiés obtenus à partir des cellules cancéreuses choisies dans différents sites sont étudiés et comparés sur des gels de séquences, et où ceux qui présentent des profils sites-spécifiques ou sites-préférentiels intéressants sont séquences et identifiés.

10. Procédé selon la revendication 9,
**caractérisé en ce que** les profils d'expression des séquences des gènes identifiés sont étudiés sur le produit obtenu à partir de tous les sites de cellules tumorales intéressants.

11. Utilisation du procédé selon la revendication 1, pour obtenir des séquences de gènes spécifiques et les produits de leur expression dans des cellules cibles présentes dans des environnements cellulaires différents ou non de ceux d'origine.
